# EUROPEAN PATENT APPLICATION

(11) **EP 2 287 768 A1**
(43) Date of publication of application: **23.02.2011**
(21) Application number: 10183851.4
(22) Date of filing: 11.02.2004
(51) Int. Cl.: G06F 19/00, A61J 1/00, G06K 9/00

(54) **Management control of pharmaceutical substances**

(30) Priority: 11.02.2003 AU 2003900586
(62) Divisional of application: 04709953.6
(71) Applicant: Pharmasea International Pty Ltd., Armadale VIC (AU)
(72) Inventor: Smith Craig, West Pymble, New South Wales 2073 (AU); Richards, Robert, Armadale, Victoria 3143 (AU)
(74) Representative: Smee, Anthony James Michael

(57) **Abstract**

A computerised identity matching management process for the supply of a pharmaceutical substance to an authorised person. The process identifies a person who is requesting the supply of the substance which includes the steps of a management computer receiving a request, from capture apparatus waiting to commence a biometric capture process representative of the person to initiate the capture process. The management computer responds to the request to return a message to the capture apparatus at a first instant in time, the message containing a unique code, and receipt of the message containing the code at the capture apparatus causes initiation of the capture process. The management computer, after returning the message, receives a captured biometric representative of the person from the capture apparatus coded with the code, at a second instant in time. The management computer operates, when the second instant is less than a predetermined time later than the first instant, to decode the captured biometric and initiate a matching process to find a match for the decoded captured biometric against stored biometric records and to retrieve an identification code representative of the person when a match is found. The management computer retrieves a date stamp and used the identification code to retrieve a data record of the person which includes at least a substance the person is prescribed, a quantity in which the substance is to be supplied and a date at which the substance is to be supplied and determines whether the date stamp matches the date at which the substance is to be supplied. If a match is determined, the substance is supplied in the prescribed quantity and information is recorded to form a record to update the supply of the substance to the person.

## Description

### Technical Field

The invention concerns a computerised identity matching management process and system for the authorised supply of a pharmaceutical substance to an individual.

### Background Art

The concept of iris recognition was developed and patented by Iridian Technologies Inc, and their concept patent US 4,641,349 describes the use of the iris to identify individuals. US 5,291,560 describes a method by which a biometric, including the iris pattern of an individual, can be used as the basis of an identification technique.

Argus Solutions Pty Ltd, developed a computerised identity matching management process and associated system. Their patent application PCT/AU02/01579 describes managing the provision of identity matching services, for instance to enable users to gain appropriate access to service provider's facilities.

### Disclosure of Invention

In a first aspect, the invention is a computerised identity matching management process for the supply of a pharmaceutical substance to an authorised person, the process comprising the steps of:
identifying a person who is requesting the supply of the substance, comprising the steps of:
   a management computer receiving a request, from capture apparatus waiting to commence a biometric capture process representative of the person to initiate the capture process;
   the management computer responding to the request to return a message to the capture apparatus at a first instant in time, the message containing a unique code, and where receipt of the message containing the code at the capture apparatus causes initiation of the capture process;
   the management computer, after returning the message, receiving a captured biometric representative of the person from the capture apparatus coded with the code, at a second instant in time; and
   the management computer operating, when the second instant is less than a predetermined time later than the first instant, to decode the captured biometric and initiate a matching process to find a match for the decoded captured biometric against stored biometric records and to retrieve an identification code representative of the person when a match is found;
   retrieving a date stamp and using the identification code to retrieve a stored data record of the person which includes at least a substance the person is prescribed, a quantity in which the substance is to be supplied and a date at which the substance is to be supplied;
   determining whether the date stamp matches the date at which the substance is to be supplied, and
   if a match is determined, supplying the substance in the prescribed quantity and recording information to form a record to update the supply of the substance to the person.

The pharmaceutical substance may be methadone, levo acetyl methadone (LAAM), buprenorphine, or the like. The authorised person to whom a substance may be supplied, may therefore be a patient. The substance may be a controlled substance used in accordance with a pharmocotherapy program. The patient may be using the substance in a therapeutic manner as a way to reduce harm associated with a dependence on mood altering chemicals.

The matching process may include generating a template image of the decoded captured biometric for matching against stored records.

The stored biometric records may include, but not be limited to, a biometric enrol template of the left iris, a biometric enrol template of the right iris, and a unique customer number. The stored biometric records may further include a portrait image of the individual. All biometric records maybe securely stored using a certified cryptographic algorithm such as 3DES. All biometric records maybe uniquely keyed to the hardware on which the database resides using a transformation process. There maybe stored biometric records for all individuals who interact with the system from groups of individuals including, but not limited to patients, doctors, clinicians, prescribers, and systems administrators.

The biometric records may be stored in a database in the management's computer cache.

The data records may be logically separate from the biometric records.

The data records may be physically separate from the biometric records.

The date stamp may be retrieved from the management computer's internal clock.

The stored data records may include, but not be limited to, any one or more of a patient database, a prescriber database, a drug register database, and a supplier database.

The patient database may include patient details, an assessment record and prescription details associated with the patient details. For instance, the patient and prescription details for each patient may include a 'name and address field', a customer identification number field which is the same number that resides in the stored biometric records, a 'create date field', a prescription 'issue date field' which indicates the date at which the prescription was prescribed, a 'prescribed drug field' to record one or more substances prescribed to the particular patient; a 'strength &/or quantity field' of each substances prescribed to the particular patient; a 'frequency of treatment field' which lists the dates, and if necessary the times, at which the each prescribed drug may be supplied to the particular patient; a 'drug supply received field' which indicates the dates on which the prescribed drug has been supplied; and a 'prescription duration field' which specifies the date at which supply of the prescribed drug is to cease.

The prescriber database may include a prescriber 'name and address field', a 'create date field' for the date on which a prescription was prescribed and who the prescription was made out to, and a customer identification number field which is the same number that resides in the stored biometric records.

The assessment database may record details of the patient including details of the patients past and current medical condition. Such an assessment may be made before any patient is prescribed medication.

The drug register database may be a governmental requirement and may be used for stock taking purposes. For example for particular types of substances such as methadone, suppliers such as pharmacists may be required by law to maintain a register of the total daily supply of the drug. Therefore, the database may include information relating to the quantity of methadone received and supplied on a daily basis, or any other time convenient or required period. The database may further include fields such as the name and address of the supplier of the substance received into stock; the name and address of the persons to whom the substance was supplied; the original prescription reference number of each person to whom the substance was supplied; the name of the prescriber; and the identity of the person making the entry.

Each of the databases may be linked so that, for example, for any patient, the prescriber or supplier records which have common fields may be easily retrieved for reference.

The method may further include the step of enrolling a person into a program so that the person is authorised to be receive the substance. In such an example, the step of enrolling the person may include:
a management computer receiving a request, from capture apparatus waiting to commence a biometric capture process representative of the person to initiate the capture process;
the management computer responding to the request to return a message to the capture apparatus at a first instant in time, the message containing a unique code, and where receipt of the message containing the code at the capture apparatus causes initiation of the capture process;
the management computer, after returning a message, receiving a captured biometric representative of the person from the capture apparatus coded with the code, at a second instant in time;
the management computer operating, when the second instant is less than a predetermined time later than the first instant, to decode the captured biometric;
generating an identification code representative of the person; and
storing a biometric record of the person's captured biometric with the identification code.

Prior to storing a biometric record of the person's captured biometric the management computer may perform a fraud check to ensure the person is not already enrolled on the system.

Having decoded the captured biometric, the management computer may transform the captured biometric into an enrolment template in order to store a record of the person's captured biometric.

Similarly, the method may include the step of identifying the prescriber who is prescribing the substance and identifying the supplier who supplies the substance to the person. The supplier may be a pharmacist.

The step of identifying the prescriber may include the steps of:
a management computer receiving a request, from capture apparatus waiting to commence a biometric capture process representative of the prescriber to initiate the capture process;
the management computer responding to the request to return a message to the capture apparatus at a first instant in time, the message containing a unique code, and where receipt of the message containing the code at the capture apparatus causes initiation of the capture process;
the management computer, after returning the message, receiving a captured biometric representative of the person from the capture apparatus coded with the code, at a second instant in time; and
the management computer operating, when the second instant is less than a predetermined time later than the first instant, to decode the captured biometric and initiate a matching process to find a match for the decoded captured biometric against stored biometric records and to retrieve an identification code representative of the prescriber when a match is found.

The step of identifying the supplier may include the steps of:
a management computer receiving a request, from capture apparatus waiting to commence a biometric capture process representative of the supplier to initiate the capture process;
the management computer responding to the request to return a message to the capture apparatus at a first instant in time, the message containing a unique code, and where receipt of the message containing the code at the capture apparatus causes initiation of the capture process;
the management computer, after returning the message, receiving a captured biometric representative of the person from the capture apparatus coded with the code, at a second instant in time; and
the management computer operating, when the second instant is less than a predetermined time later than the first instant, to decode the captured biometric and initiate a matching process to find a match for the decoded captured biometric against stored biometric records and to retrieve an identification code representative of the supplier when a match is found.

In a second aspect the invention is a computerised identity matching management system for the authorised supply of a pharmaceutical substance to an authorised person, comprising:
a data depository for storing records which include, for each person, at least the substance a person is prescribed, the quantity in which the substance is to be supplied and the date at which the substance is to be supplied;
a computer programmed to:
   receive a request, from capture apparatus waiting to commence a biometric capture process, to initiate the capture process to identify a person who is requesting the supply of a substance;
   respond to the request to return a message to the capture apparatus at a first instant in time, the message containing a unique code, and where receipt of the message containing the code at the capture apparatus causes initiation of the capture process;
   after returning the message, receiving a captured biometric from the capture apparatus coded with the code, at a second instant in time; and
   when the second instant is less than a predetermined time later than the first instant, to decode the captured biometric;
   an authentication server to perform a matching process to find a match for the decoded captured biometric against stored biometric records and to retrieve the identification code representative of the person who is requesting the supply of a pharmaceutical substance when a match is found, the server further retrieving a date stamp and using the identification code to retrieve the person's stored data record to determine whether the date stamp matches the date at which the substance is to be supplied, and if a match is determined updating the person's stored data record relating to the supply of the substance in the prescribed quantity to the person.

There are some forms of medication, for example methadone, the delivery of which is strictly controlled. Generally it is the government that places strict controls on the delivery of such products and the onus is on the dispenser to ensure that the product is only supplied to persons who are entitled to receive it, is only delivered in the dose that is prescribed, and is only delivered at pre-determined intervals. A further constraint on the dispenser is the maintenance of dose changes, prescription expiry and general patient management issues. Furthermore, for many pharmacists, the delivery of methadone therapy to former drug addicts is a time consuming and very stressful part of their business because of the tight constraints, which are placed upon them and the possible adverse results to the recipient if an incorrect drug or incorrect quantity is delivered, or if a drug is delivered to the person more frequently that prescribed.

It is therefore, an advantage of at least one example of the invention that pharmaceutical substances are supplied to authorised persons in the correct doses and correct time periods.

A further advantage of at least one embodiment of the invention is that the individual's transformed biometric data and the individual's patient data is logically separate. A further advantage of at least one embodiment of the invention is that the identification of an individual is completed via an anonymous procedure. No information other than the captured biometric is required to perform a search of all enrolled persons.

### Brief Description of Drawings

An example of the system will now be described with reference to the accompanying drawings; in which:
Fig. 1 is a schematic diagram of a system used to manage the supply of a pharmaceutical substance to a patient; and
Fig. 2 is a flow chart showing the steps a patient seeks the supply of a substance.

### Best Mode for Carrying Out the Invention

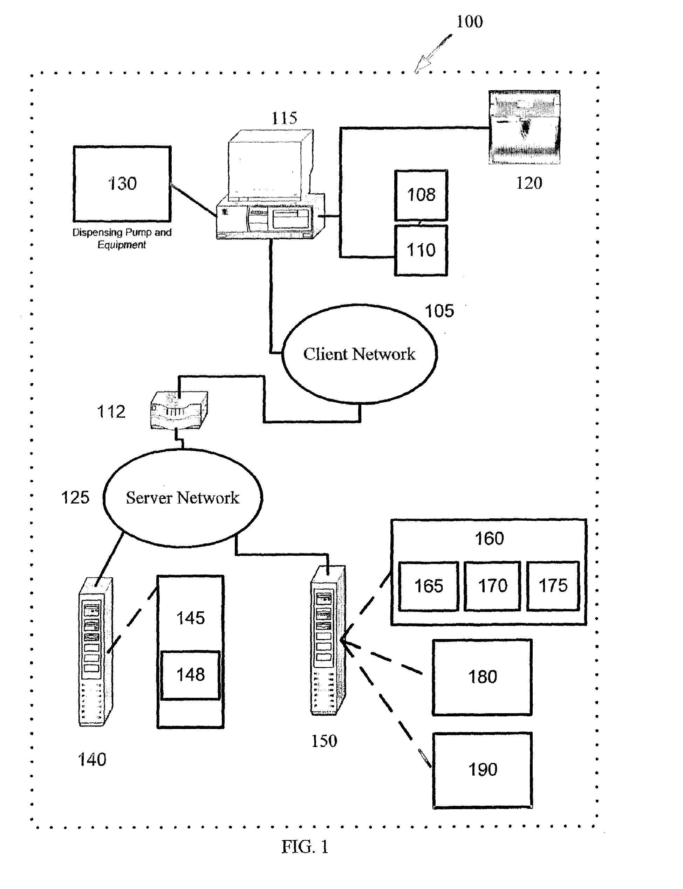

Fig. 1 illustrates a system 100 used to manage the authorised supply of Methadone to patients and to ensure that the right patient get the prescribed dose at the prescribed time. The system 100 includes an Iris Recognition client computer 115 which is programmed to receive and transmit messages through a firewall 112 and over the Internet using the client software 108. The software 108 resides in the PC 115. The software 108 works with PrivatelD software 110 and an iris recognition camera 120 such as the Panasonic BM-ET300 which includes a special lenses to photograph the eyes. The Authentication server 140 accepts the iris image(s) which is(are) sent from the client software 108. Prior to sending, the client software 108 ensures that the images are of sufficient quality for processing by the Authentication Server 140. In addition, the server 140 confirms the image integrity and then sends it through the iris recognition process for verification against biometric records stored in its cache 145 which in turn is drawn from the secure database 148.

The Records Database Server 150 stores a patient database 160, a prescriber database 180 and a drug register database 190. The patient database 160 include three sub records; patient details 165, an assessment record 170 and prescription details 175.

The patient and prescription details for each patient include:
- a 'name and address field'
- a Customer Identification Number field (this number is the same number that resides in the customers record within the Authentication Server 140. It is linked to their biometric templates),
- a 'create date field'
- a prescription 'issue date field' which indicates the date at which the prescription was prescribed. This field is important as the duration period of some controlled substances is determined by the date on which they were prescribed;
- a prescription registration number
- a 'prescribed drug field', to record one or more substances prescribed to the particular patient;
- a length &/or quantity field' of each substance prescribed to the particular patient;
- a 'frequency of treatment field' which lists the dates, and if necessary the times, at which the each prescribed drug may be supplied to the particular patient;
- a 'drug supply received field' which indicates the dates on which the prescribed drug has been supplied; and
- a ' prescription duration field' which specifies the date at which supply of the prescribed drug is to cease. This date corresponds to the last entry in the frequency of treatment field.

The assessment record 170 is created each time a new patient is referred to a prescriber for participation on the Methadone program.

The prescriber database 180 includes:
- a 'name and address field' of the prescriber
- Customer Identification Number field (this number is the same number that resides in the customers record within the Authentication Server 140. It is linked to their biometric templates for circumstances where the prescriber is required to use iris recognition;
- and a 'create date field' for when the prescriber prescribes a prescription.

The patient 165, assessment 170 and prescription 175 records are linked for each patient.

The drug register database 190 includes the following fields:
- quantity of methadone received;
- the name and address of the supplier of the methadone received into stock;
- daily quantity of methadone supplied;
- the name and address of the persons to whom methadone was supplied by the pharmacy;
- the original prescription reference number of each person to whom methadone was supplied;
- the name of the prescriber;
- the balance of methadone in stock after each transaction;
- the identity of the person making the entry.

In this example, some of the components of the system 100, namely the PC 115, software 108, 110 and the Iris recognition camera 120 are installed on the client network 105, which could be a pharmacy or a clinic and the premises of a prescriber. Other components of the system 100, namely the Authentication Server 140, its cache 145 and the records server 150 with its associated databases, are installed on a server network 125 remote from the client network 105.

With regard to the pharmacists/clinical facilities, the PC 115 is accessed by pharmacists or operators who have the authorisation to dispense or supply methadone to patients. The camera 120 is used to at least capture an iris image of a patient who presents themselves at the pharmacy for the supply of methadone.

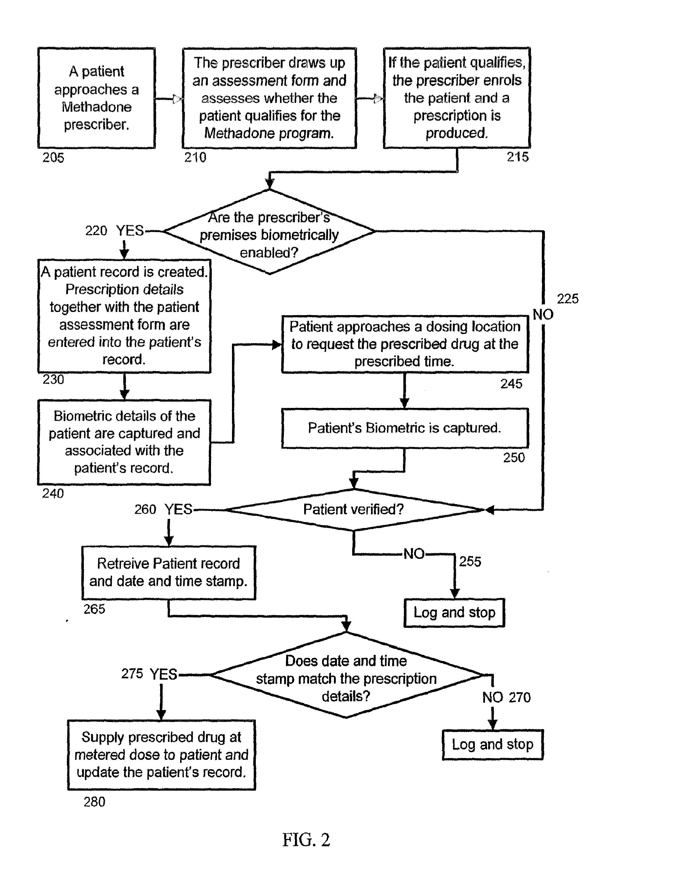

Fig. 2 illustrates a flow chart which indicates the steps undertaken from when a patient approaches a Methadone prescriber, step 205, to when the prescribed methadone is dispensed to the patient, step 280. After a patient has approached the prescriber, the prescriber draws up an assessment form and assesses whether the patient qualifies for the methadone program, step 210. If the patient qualifies, then the prescriber proceeds to enrol the patient and a prescription is produced, or a referral to an authorised person (ie: a doctor) so that a prescription can be produced 215. The prescription includes details of the patient and the quantity and frequency of Methadone to be supplied to that particular patient. If the premises of the prescriber are not biometrically enabled, step 225 then the patient is required to take the prescription to a pharmacy at the required time 245 whereby the patient is verified in a traditional manner.

It is also possible to have an authorised operator scan in a hard copy of a prescription from another authorised source (eg: the patient's local doctor).

If the prescribers premises are biometrically enabled, step 220, then the step of enrolling the patient involves creating a record of the patient in the patient database 160 of the record server 150 and of creating an enrolment record in the biometric database 148 of the Authentication Server 140. At step 230, prescription details 175 together with the patient's assessment form 170 are captured and are entered into the appropriate records of the patient's database 160. The biometric details of the patient are then captured, step 240.

### Enrolling the Patient

Step (a). The client software 108 is launched and captures the Private ID software 110 to take control of the camera 120 so as to record an image of the patients right and left irises.

Step (b). The client software 108 sends a message to the client computer 105 for a message authentication code (MAC). The client 105 responds to the request and issues a MAC. The client software 108 receives the MAC and the Private ID software 110 commences capture of the patients iris.

Step (c). To use the Panasonic BM-ET300 camera 120, the patient moves their head so that they can see both of their eyes in the mirror in the centre of the camera head at a distance of approximately 43 to 48 cm (17 to 19 inches) from the lens. The camera 120 sends images to the software 108 running on the computer 115.

Step (d). The PrivateID software 110 captures a series of digital video images of the patients eyes. Image quality metrics within the client software 108 inspect the images for sufficient quality and iris content to ensure high confidence for a successful match outcome. Once a satisfactory images have been culled, the software 108 provides a feedback signal to inform that the image capture session is complete, this usually issues within seconds. If a satisfactory image cannot be captured within the allotted time (the default is set at 10 seconds), then the software provides an error signal. The patient would then have to restart the process of having images of the iris captured.

Step (e). Once captured, the process of creating an iriscode templates for the patient begins. The PrivateID software 110 encrypts the captured image using an appropriate cryptographic algorithm. Then it compresses the captured image, codes the compressed image using the previously issued MAC and assembles a message for transmission to the client computer 105. This transmission is handled by client software 108.

Step (f). The authentication server 140 receives the message and checks it for validity using MAC, that is to ensure it has been received while the MAC is still valid. The message then has its integrity checked using a checksum, and is decompressed and decrypted. It is then passed through a Daugman Algorithm, or similar, to create the iriscodes.

Step (g). The iriscodes are then sent to the authentication server cache 145 to ensure that the records do not already exist (Fraud Check). Providing the records do not already exist, the IrisCode templates and unique customer identification record (and optional portrait image) are entered into the database 148 and the cache 145 is updated. The Authentication Server 140 ensures that the unique customer identification number entered into the database 148 is correctly associated with the appropriate patient record 165 in database 160.

### Biometric Capture for supply of Methadone

At some later date, the patient approaches a pharmacy to request the supply of the methadone, step 245. The patient is required to be identified by iris recognition and proceeds to have their biometric captured, step 250.

Steps (a) to (f) of the patient admission are repeated. However step (g) in this circumstance is different. The recognition iriscode templates are sent to the authentication server 140 which attempts to match it against all records in its secure cache 145. The authentication server 140 returns a result to the client computer 105 which interprets the result. If the result is a comparison failure, that result is logged and the process stops, step 255. The pharmacist checks the frequency of the treatment field to determine when the drug is prescribed to be supplied. The patient is informed of this date.

If a match is found, step 260, the result indicates that the patient has been identified and the authentication server retrieves the relevant patient record data step 265, and returns to the requesting application 108. If the patient is authorised to receive the prescribed supply of methadone step 275, the pharmacist checks the quantity and strength of the drug and accordingly dispenses to the patient step 280. Should the clinic/pharmacy be equipped with an automated dispensing platform, this will then be used to dispense the correct amount of Methadone as required 130. The prescribed supply will be administered or packaged according to the information on the prescription. The supply of the drug is recorded in the 'drug supply field' of the patients record 160.

If the date and time stamp does not match the prescription details 270, the pharmacist checks the frequency of the treatment field to determine when the drug is prescribed to be supplied. The patient is informed of this date. The patient details 165 are updated to reflect the occurrence, step 270.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. A computerised identity matching management process for the supply of a pharmaceutical substance to an authorised person, the process comprising the steps of:
identifying a person who is requesting the supply of the substance, comprising the steps of:
a management computer (140) receiving a request, from capture apparatus (120) waiting to commence a biometric capture process representative of the person to initiate the capture process;
the management computer (140) responding to the request to return a message to the capture apparatus (120) at a first instant in time, the message containing a unique code, and where receipt of the message containing the code at the capture apparatus causes initiation of the capture process;
the management computer, after returning the message, receiving a captured biometric representative of the person from the capture apparatus coded with the unique code, at a second instant in time; and
the management computer operating, when the second instant is less than a predetermined time later than the first instant, to decode the captured biometric and initiate a matching process to find a match for the decoded captured biometric against stored biometric records (148) and to retrieve an identification code representative of the person when a match is found;
retrieving a date stamp and using the identification code to retrieve a stored data record (175) of the person which includes at least a substance the person is prescribed, a quantity in which the substance is to be supplied and a date at which the substance is to be supplied, wherein the stored data records (160, 180, 190) are logically separate from the biometric records (148);
determining whether the date stamp matches the date at which the substance is to be supplied, and
if a match is determined, supplying the substance in the prescribed quantity and recording information to form a record to update the supply of the substance to the person.

2. The process according to claim 1, where the biometric records (148) are securely stored within the management computer's cache (145).

3. The process according to any one of the preceding claims, where the stored data records (160) are physically separate from the biometric records.

4. The process according to any one of the preceding claims, where the matching process includes generating a template image of the decoded captured biometric for matching against stored biometric records (148).

5. The process according to claim 4, where the stored biometric records (148) include a biometric enrol template of the left iris, a biometric enrol template of the right iris, and the identification code.

6. The process according to claim 5, where the stored biometric records further include a portrait image of the person.

7. The process according to any one of the preceding claims, where the stored data records include a patient database (160) and a prescriber database (180).

8. The process according to claim 7, where the stored data records further include a drug register database and a supplier database.

9. The process according to any one of the preceding claims, further including the step of enrolling a person into a program so that the person is authorised to receive the substance.

10. The process according to claim 9, where the step of enrolling the person includes:
a management computer (140) receiving a request, from capture apparatus (120) waiting to commence a biometric capture process representative of the person to initiate the capture process;
the management computer responding to the request to return a message to the capture apparatus at a first instant in time, the message containing a unique code, and where receipt of the message containing the code at the capture apparatus causes initiation of the capture process;
the management computer, after returning a message, receiving a captured biometric representative of the person from the capture apparatus coded with the unique code, at a second instant in time;
the management computer operating, when the second instant is less than a predetermined time later than the first instant, to decode the captured biometric;
generating an identification code representative of the person; and storing a biometric record of the person's captured biometric with the identification code.

11. The process according to claim 10, wherein prior to storing a biometric record of the captured biometric, the management computer (140) performing a fraud check to ensure the person is not already enrolled on the system.

12. The process according to claim 10 or claim 11, wherein having decoded the captured biometric, the management computer transforming the captured biometric into an enrolment template in order to store a biometric record of the person's captured biometric

13. The process according to any one of the preceding claims, where the predetermined time is determined according to the time required for the biometric capture process.

14. The process according to any one of the preceding claims, where the substance is a controlled substance.

15. The process according to any one of the preceding claims, where the method further includes the step of identifying a prescriber who is prescribing the substance comprising the steps of:
a management computer (140) receiving a request, from capture apparatus (120) waiting to commence a biometric capture process representative of the prescriber to initiate the capture process;
the management computer responding to the request to return a message to the capture apparatus at a first instant in time, the message containing a unique code, and where receipt of the message containing the code at the capture apparatus causes initiation of the capture process;
the management computer, after returning the message, receiving a captured biometric representative of the person from the capture apparatus coded with the unique code, at a second instant in time; and
the management computer operating, when the second instant is less than a predetermined time later than the first instant, to decode the captured biometric and initiate a matching process to find a match for the decoded captured biometric against stored biometric records and to retrieve an identification code representative of the prescriber when a match is found.

16. The process according to any one of the preceding claims, where the method further includes the step of identifying a supplier who supplies the substance to the person comprising the steps of:
a management computer (140) receiving a request, from capture apparatus (120) waiting to commence a biometric capture process representative of the supplier to initiate the capture process;
the management computer responding to the request to return a message to the capture apparatus at a first instant in time, the message containing a unique code, and where receipt of the message containing the code at the capture apparatus causes initiation of the capture process;
the management computer, after returning the message, receiving a captured biometric representative of the person from the capture apparatus coded with the code, at a second instant in time; and
the management computer operating, when the second instant is less than a predetermined time later than the first instant, to decode the captured biometric and initiate a matching process to find a match for the decoded captured biometric against stored biometric records and to retrieve an identification code representative of the supplier when a match is found.

17. A computerised identity matching management system for the authorised supply of a pharmaceutical substance to an authorised person, comprising:
a data depository (160) for storing data records which include, for each person, at least the substance a person is prescribed, the quantity in which the substance is to be supplied and the date at which the substance is to be supplied,
a management computer (140) programmed to:
receive a request, from capture apparatus waiting to commence a biometric capture process, to initiate the capture process to identify a person who is requesting the supply of a substance;
respond to the request to return a message to the capture apparatus at a first instant in time, the message containing a unique code, and where receipt of the message containing the code at the capture apparatus causes initiation of the capture process;
after returning the message, receiving a captured biometric from the capture apparatus coded with the unique code, at a second instant in time; and when the second instant is less than a predetermined time later than the first instant, to decode the captured biometric;
an authentication server to perform a matching process to find a match for the decoded captured biometric against stored biometric records and to retrieve the identification code representative of the person who is requesting the supply of a pharmaceutical substance when a match is found, the server further retrieving a date stamp and using the identification code to retrieve the person's stored data record to determine whether the date stamp matches the date at which the substance is to be supplied, and if a match is determined updating the person's stored data record relating to the supply of the substance in the prescribed quantity to the person, where the data records stored in the data depository (160) are logically separate from the biometric records (148).

18. The system according to claim 17, where the data records stored in the data depository (160) are physically separate from the biometric records (148) which are stored within the management computer's cache.

19. The system according to any one of claims 17 or 18, where the stored data records include a patient database (160) and a prescriber database (180),

20. The system according to claim 19, where the stored data records further include a drug register database and a supplier database.

21. The system according to any one of claims 17 to 20, further comprising a firewall 112 between the management computer and at least the capture apparatus.
